# EUROPEAN PATENT SPECIFICATION

(11) **EP 3 917 378 B1**
(45) Date of publication and mention of the grant of the patent: **08.01.2025**
(21) Application number: 19912286.2
(22) Date of filing: 27.08.2019
(51) Int. Cl.: A61B 5/00, G01N 33/50, G01N 33/68

(54) **SYSTEMS AND METHODS FOR HOME TRANSDERMAL GFR MONITORING**
SYSTEME UND VERFAHREN ZUR HÄUSLICHEN TRANSDERMALEN GFR-ÜBERWACHUNG
SYSTÈMES ET PROCÉDÉS DE SURVEILLANCE TRANSDERMIQUE DU DFG À DOMICILE

(30) Priority: 28.01.2019 US 201962797543 P
(43) Date of publication of application: 08.12.2021
(73) Proprietor: MediBeacon Inc., St. Louis MI 63141 (US)
(72) Inventor: DORSHOW, Richard, B., St. Louis, Missouri 63132 (US); HANLEY, Steven, J., St. Louis, Missouri 63132 (US); STERN, Terrence, St. Louis, Missouri 63132 (US)
(74) Representative: Deambrogi, Edgardo
(86) International application number: PCT/US2019/048307
(87) International publication number: WO 2020/159578

(56) References cited:
- WO-A1-2015/153860
- US-A- 5 226 417
- US-A1- 2011 230 739
- US-A1- 2011 230 739
- US-A1- 2013 303 865
- US-A1- 2013 303 865
- US-A1- 2016 001 000
- US-B2- 8 591 865
- CARDIO CRITIC: "Kardia Mobile Review - ECG (EKG) Machine - smartphone connected", YOUTUBE, 14 February 2017 (2017-02-14), XP054980825, Retrieved from the Internet <URL:https://www.youtube.com/watch?v=1GHR-tWU_S4> [retrieved on 20191009]
- EXCELLENT WEBWORLD: "Best Pulse Oximeter app for iPhone & Android: Connected Medical Devices Monitor App Demo", YOUTUBE, 10 October 2017 (2017-10-10), XP054980823, Retrieved from the Internet <URL:https://www.youtube.com/watch?v=1m-jniZo-Jg> [retrieved on 20191009]

## Description

### FIELD OF INVENTION

The field of the disclosure relates generally to a computer program and system for facilitating home medical care. More specifically, this disclosure relates to monitoring of the glomerular filtration rate (GFR) in a patient outside of a clinical or hospital setting using a mobile computing device and a system for performing the same.

### BACKGROUND

In the clinical and preclinical field, determining various organ functions is accorded great importance since, for example, corresponding therapies or medications can be controlled in accordance with said organ functions.

The glomerular filtration rate (GFR) is an important clinical parameter to assess the level of kidney function in a patient. As shown in the table below, the lower the GFR, the more serious the kidney impairment for Chronic Kidney Disease (CKD) and other renal insufficiencies. The GFR can be estimated based on a blood test measuring the blood creatinine level in the patient in combination with other factors. More accurate methods involve the injection of an exogenous substance into a patient followed by careful monitoring of plasma and/or urine concentration over a period of time. These are often contrast agents (CA) that can cause renal problems on their own. Radioisotopes or iodinated aromatic rings are two common categories of CAs that are used for GFR determination.

| Stage | Description | GFR* |
|---|---|---|
| Increased risk | Increase of risk factors (e.g., diabetes, high blood pressure, family history, age, ethnicity) | > 90 |
| 1 | Kidney damage with normal kidney function | > 90 |
| 2 | Kidney damage with mild loss of kidney function | 60-89 |
| 3a | Mild to moderate loss of kidney function | 44-59 |
| 3b | Moderate to severe loss of kidney function | 30-44 |
| 4 | Severe loss of kidney function | 15-29 |
| 5 | Kidney failure; dialysis required | < 15 |

| | | |
|---|---|---|
| * GFR is measured in units of mL/min/1.73m². | | |

Many patients with impaired kidney function also suffer from numerous other medical difficulties and may have limited or impaired mobility. In some instances, patients are homebound and receiving medical care from a home health provider such as a nurse. It can be difficult for these patients to travel to a hospital or clinical location for medical assessment. The patients most in need of accurate and timely assessment may have the greatest difficulty in getting that assessment. Thus, there is a need for assessing the GFR of a patient without requiring the patient to travel to a hospital or clinical location for the assessment. US 2011/230739 discloses a sensor plaster for the transcutaneous measurement of an organ function, e.g. he glomerular filtration rate.

### BRIEF DESCRIPTION

In one aspect, disclosed herein is a non-transitory computer-readable media having computer-executable instructions thereon, according to claim 1.

In another aspect, disclosed herein is a computer-implemented method for assessing the GFR in a patient, according to claim 5.

Disclosed herein is a system for assessing the GFR in a patient. The system generally includes a mobile computing device having installed thereon a computer program for assisting a user in assessing the GFR in the patient, a GFR sensor configured to be wirelessly communicatively coupled to the mobile computing device, a GFR agent, and an injector device for the GFR agent.

In yet another aspect, disclosed herein is a kit for GFR assessment, according to claim 11.

### BRIEF DESCRIPTION OF THE DRAWINGS

The Figures described herein depict various aspects of the systems and methods disclosed therein. It should be understood that each Figure depicts an embodiment of a particular aspect of the disclosed systems and methods, and that each of the Figures is intended to accord with a possible embodiment thereof.
Figure 1 is an example screenshot of a home screen for a software application used to assess the GFR in a patient in accordance with the present disclosure.
Figure 2 is an example screenshot of a Bluetooth pairing screen for the software application that instructs the patient to communicatively couple a mobile computing device with a GFR sensor in accordance with the present disclosure.
Figure 3 is an example screenshot of a sensor placement screen for the software application that instructs the patient how and where to place the sensor on their body.
Figure 4 is an example screenshot of a customization screen for the software application that instructs the patient to customize the sensor to a skin tone or other physiological characteristic of the patient.
Figure 5 is an example screenshot of an injection screen for the software application that instructs the patient to inject themselves with a GFR agent.
Figure 6 is an example screenshot of a measurement screen for the patient application that provides feedback to the patient while the GFR is being measured.
Figure 7 is an example screenshot of a results screen for the software application that displays a final GFR, and enables the patient to transmit the final GFR to his or her health care provider.
Figure 8 is an example screenshot of an exit screen for the software application that enables the patient to exit the software application.
Figure 9 is a block diagram of one embodiment of a computing device that may be used with the system shown in Figure 10.
Figure 10 is a block diagram of one embodiment of a system for home transdermal GFR monitoring.

### DETAILED DESCRIPTION

Approximating language, as used herein throughout the specification and claims, may be applied to modify any quantitative representation that could permissibly vary without resulting in a change in the basic function to which it is related. Accordingly, a value modified by a term or terms, such as "about," "approximately," and "substantially," are not to be limited to the precise value specified. In at least some instances, the approximating language may correspond to the precision of an instrument for measuring the value. Here and throughout the specification and claims, range limitations may be combined and/or interchanged; such ranges are identified and include all the sub-ranges contained therein unless context or language indicates otherwise.

The computer-implemented methods discussed herein may include additional, less, or alternate actions, including those discussed elsewhere herein. The methods may be implemented via one or more local or remote processors, transceivers, servers, and/or sensors, and/or via computer-executable instructions stored on non-transitory computer-readable media or medium.

Additionally, the computer systems discussed herein may include additional, less, or alternate functionality, including that discussed elsewhere herein. The computer systems discussed herein may include or be implemented via computer-executable instructions stored on non-transitory computer-readable media or medium.

As will be appreciated based upon the specification, the described embodiments of the disclosure may be implemented using computer programming or engineering techniques including computer software, firmware, hardware or any combination or subset thereof. Any such resulting program, having computer-readable code means, may be embodied or provided within one or more computer-readable media, thereby making a computer program product, e.g., an article of manufacture, according to the discussed embodiments of the disclosure. The computer-readable media may be, for example, but is not limited to, a fixed (hard) drive, diskette, optical disk, magnetic tape, semiconductor memory such as read-only memory (ROM), and/or any transmitting/receiving medium, such as the Internet or other communication network or link.

These computer programs (also known as programs, software, software applications, "apps", or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The "machine-readable medium" and "computer-readable medium," however, do not include transitory signals. The term "machine-readable signal" refers to any signal used to provide machine instructions and/or data to a programmable processor.

As used herein, a processor may include any programmable system including systems using micro-controllers, reduced instruction set circuits (RISC), application specific integrated circuits (ASICs), logic circuits, and any other circuit or processor capable of executing the functions described herein. The above examples are example only, and are thus not intended to limit in any way the definition and/or meaning of the term "processor."

As used herein, the terms "software" and "firmware" are interchangeable, and include any computer program stored in memory for execution by a processor, including RAM memory, ROM memory, EPROM memory, EEPROM memory, and non-volatile RAM (NVRAM) memory. The above memory types are example only, and are thus not limiting as to the types of memory usable for storage of a computer program.

In one aspect, a computer program (i.e., a software application) is provided, and the program is embodied on a computer readable medium. In an exemplary embodiment, the program is executed on a single computer system, without requiring a connection to a server computer. In a further embodiment, the computer system is a mobile computing device such as a smartphone or a tablet. The computer program is flexible and designed to run in various different environments without compromising any major functionality. Nonlimiting examples of different computing environments include smartphones running either the iOS or Android operating systems.

A computer program (i.e., a software application installed on a non-transitory computer-readable medium) and system are described hereinafter substantially with regard to kidney function monitoring. In principle, however, other applications are also conceivable in which the function of a particular organ can be detected by means of determining a temporal profile of an indicator substance.

At a high level, provided herein is a computer program and a sensor configured to attach to the skin of a patient. The computer program is designed to run on a mobile computing device and the sensor (also referred to herein as a GFR sensor) is designed to be in wireless communication with the computer program (e.g., via a Bluetooth connection). The patient attaches the sensor to their body, e.g., on the skin, and, after the sensor is calibrated and customized to the patient's unique skin tone and physiological characteristics, the patient then injects him or herself with a GFR agent using an injector device. The GFR agent is configured to emit light in response to electromagnetic radiation emitted by the sensor. A detector on the sensor then detects the emitted light over a period of time and transmits information about the detected light to the mobile computing device running the computer program. Information transmitted can include, but is not limited to, detected light intensity and time. This information is then used by the computer program to assess the GFR of the patient. The GFR is then either displayed on an interface of the mobile computing device, and in some aspects, the GFR is additionally transmitted from the mobile computing device to a health care provider of the patient. This system for assessing the GFR of the patient is designed to be operated outside of a hospital or health care provider's office. For example, it may be operated by the patient in their own residence.

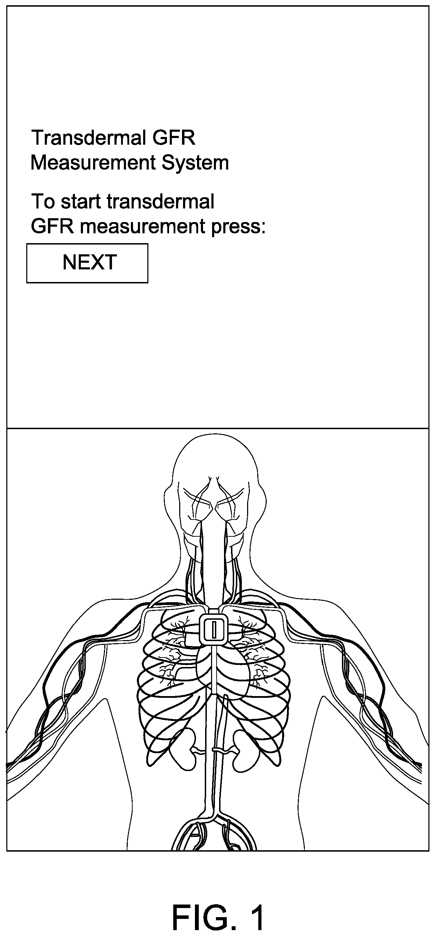

Figures 1 to 8 illustrate example screen shots of a user interface of the software application in accordance with the example embodiments of the present disclosure. The screen shots are displayed on a mobile computing device executing the software application. More specifically, Figure 1 is an example screenshot of a home screen displayed on the mobile computing device. In the example embodiment, the home screen is the first screen displayed to the user when accessing the software application on the mobile computing device. The home screen instructs the user to proceed to the next step. Additional functionality, not show, can be incorporated on this screen or on additional screens of the program. Additional functionality can include, but is not limited to, information such as patient identification, insurance information, contact information for the patient or the patient's health care provider (HCP). This, or any additional screen of the application, can also include a "Help" functionality in order to further instruct the patient or answer questions with respect to the operation and results of the procedure.

In the exemplary embodiment, the patient (for whom the GFR measurement is to be obtained) accesses and uses the software application on the mobile computing device. Alternatively, a user other than the patient (e.g., an in-home care giver) may access and use the software application to obtain a GFR measurement for the patient.

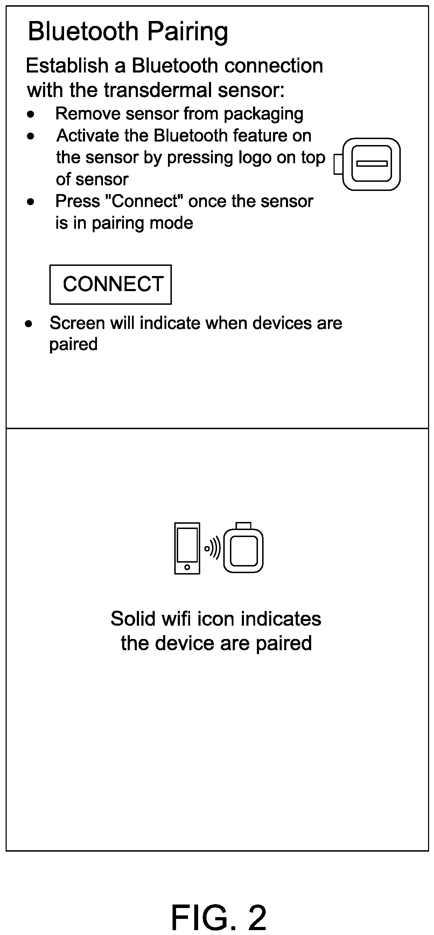

Figure 2 is an example screenshot of a Bluetooth pairing screen for the software application. As shown in Figure 2, the Bluetooth pairing screen instructs the patient (or other user) to "pair" or communicatively couple the mobile computing device to a GFR sensor. Pairing can be done using, for example, Bluetooth and/or any other suitable form of wireless communication between the mobile computing device and the GFR sensor. Pairing can incorporate security features, such as requiring the user to enter a unique sensor identification number into the software application (e.g., using an input interface of the mobile computing device) to enable the pairing. In some aspects, no identification is required instead relying on the limited range of Bluetooth technology and proximity of the sensor to the mobile computing device.

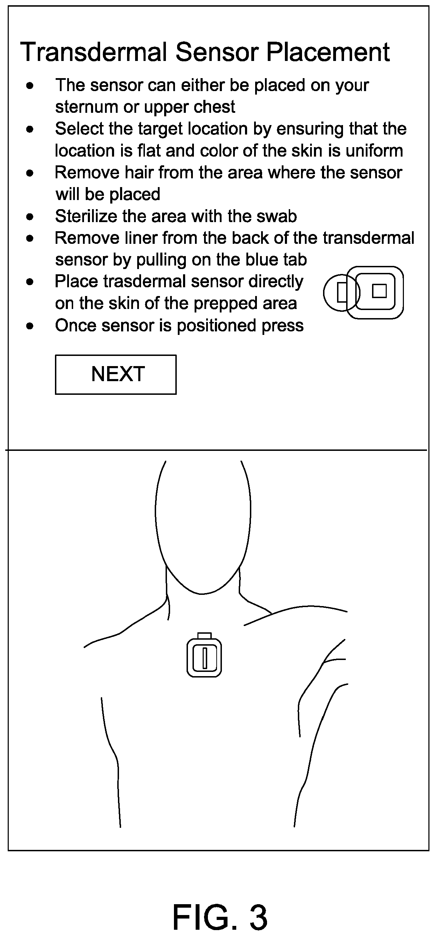

Figure 3 is an example screenshot of a sensor placement screen for the software application. As shown in Figure 3, the sensor placement screen instructs the patient to place the GFR sensor on their skin. The GFR sensor can be placed in any suitable location on the body of the patient, although, in some aspects, the patient is instructed to place the GFR sensor in a specific spot. Additional instructions can be include with this, or an additional, screen of the computer program. In this example, the patient is also instructed regarding how to select a location on their skin for the GFR sensor, and how to prepare and sterilize the location in order to achieve an optimal attachment of the GFR sensor. Additionally, the screen also includes a selectable icon (e.g., a button) for the patient to confirm that the GFR sensor has been placed properly on their skin. This prevents the computer program from moving on to the next step of the procedure until the GFR sensor has been placed properly. Additional functionality can be incorporated into this, or any additional, screen of the computer program. In some aspects, the instructions illustrated in Figure 3 are split between two or more screens.

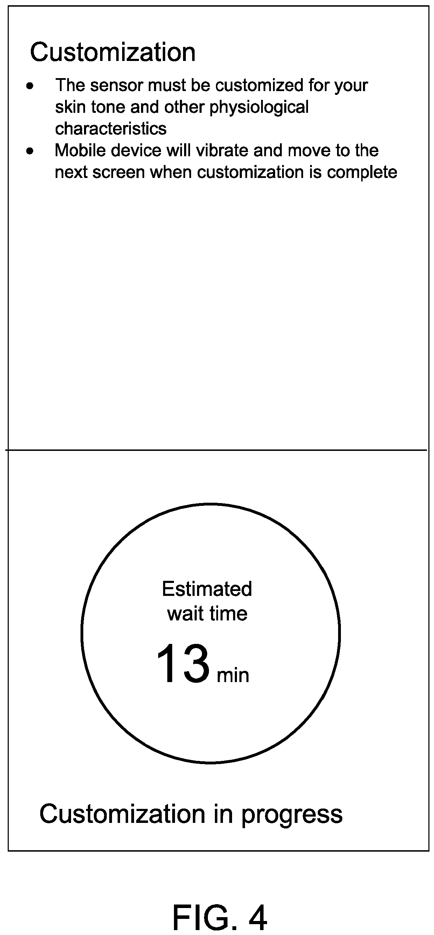

Figure 4 is an example screenshot of a customization screen for the software application. As shown in Figure 4, the customization screen instructs the patient to customize the GFR sensor to the patient's own skin tone and/or other physiological characteristics. In this aspect, the GFR sensor receives a signal from the mobile computing device instructing the GFR sensor to initiate a customization process. The customization process may include collecting a background level of light absorbance that naturally emanates from the body of the patient. It may also include adjusting the light strength or detector sensitivity in the sensor to account for differing skin colors of different patients. For example, patients of different ethnic background may have different skin tones, and the sensor adjusts to account for the different skin tones. In some aspects, the customization screen includes a countdown timer informing the patient as to how long before customization is complete and the patient can proceed to the next step in the procedure.

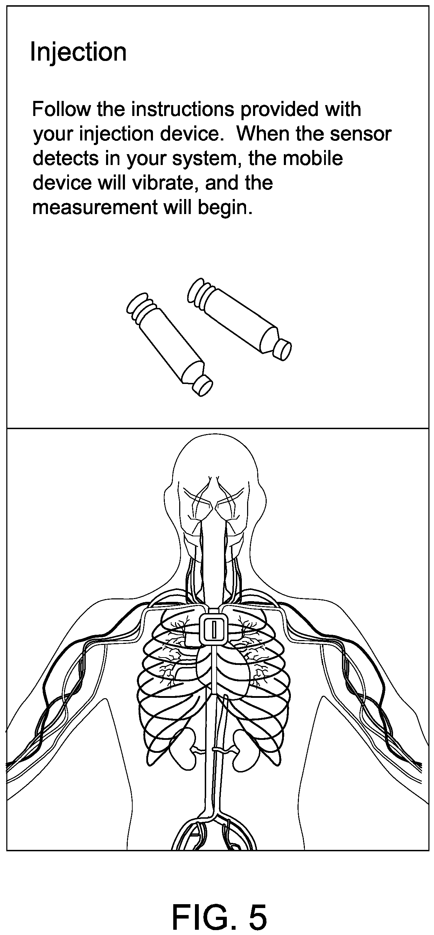

Figure 5 is an example screenshot of an injection screen for the software application. As shown in Figure 5, the injection screen instructs the patient to inject him or herself with a GFR agent detectable by the GFR sensor using an injector device. In some aspects, the patient is instructed specifically where on their body to inject the GFR agent. For example, a patient may be instructed to inject the GFR agent into their leg, abdomen or buttocks. The injector device comprises an injector system and a dose of the GFR agent. The dose of the GFR agent may be preloaded into the device or the patient may be provided with instructions how to load the dose into the device. In this aspect, the patient is also instructed that the sensor will vibrate when it detects the presence of the GFR agent inside the body of the patient. This informs the patient that the measurement has begun and that the GFR agent was properly injected.

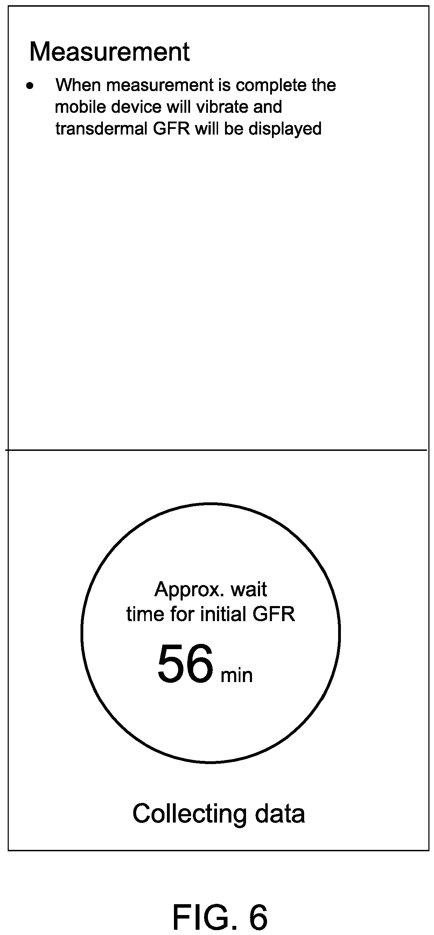

Figure 6 is an example screenshot of a measurement screen for the patient application. As shown in Figure 6, the measurement of the GFR of the patient by the GFR sensor proceeds without any additional action by the patient. In this aspect, the patient is informed that a predetermined amount of time must pass before the assessment is complete. Additionally, the computer program is configured such that the mobile computing device wirelessly receives data from the GFR sensor during the assessment. In some aspects, the mobile computing device receives light absorbance data from the GFR sensor, and the computer program uses the received light absorbance data to calculate the GFR of the patient using mathematical algorithms included in the software application. One such algorithm is described in US Patent Application 16/171,689 filed on October 26, 2018. For the measurement, the HCP of the patient may provide additional instructions as to what activities may or may not be permitted during the assessment. For example, the patient may be instructed that showering, bathing or strenuous exercise during the assessment is not permitted, but other routine activities are permitted. In some aspects, the computer program calculates an "initial" GFR that is displayed on the screen of the mobile computing device part way through the assessment. This can be used to ensure that the sensor is properly collecting data while recognizing that light absorbance data collected over a longer period of time may be a more accurate assessment of the GFR of the patient.

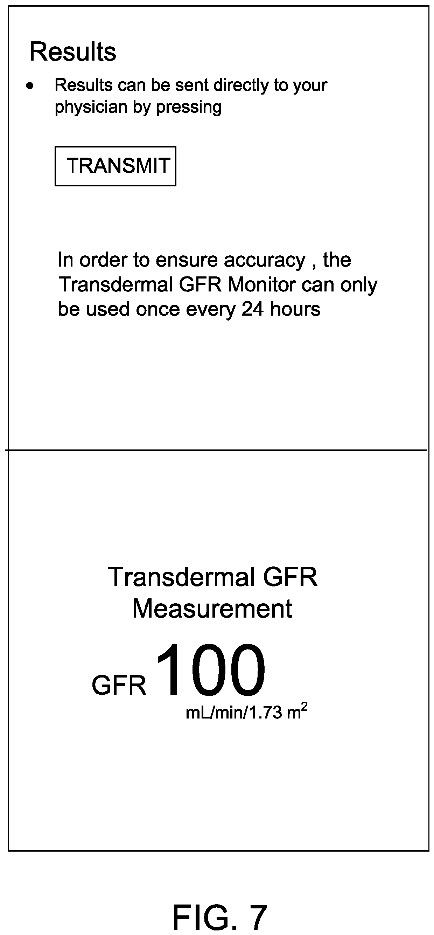

Figure 7 is an example screenshot of a results screen for the software application. As shown in Figure 7, the final results of the GFR assessment are displayed on the screen of the mobile computing device. In some aspects, the patient is given the option to transmit the final results of the assessment from the mobile computing device to an HCP computing device associated with the HCP (e.g., by selecting a "TRANSMIT" button), while in other aspects, the computer program automatically causes the final results to be transmitted from the mobile computing device to the HCP computing device. Transmission of the results can be done using methods know in the art for transmitting data from one computing device to another. In the simplest example, the results can be transmitted by email from the patient's mobile computing device to the computing device of the HCP. Other transmission methods may also be used. In all aspects, because this is patient medical information, compliance with all laws regarding patient privacy is incorporated into this transmission. For example, the data may be encrypted before transmission from the mobile computing device to the computing device of the HCP.

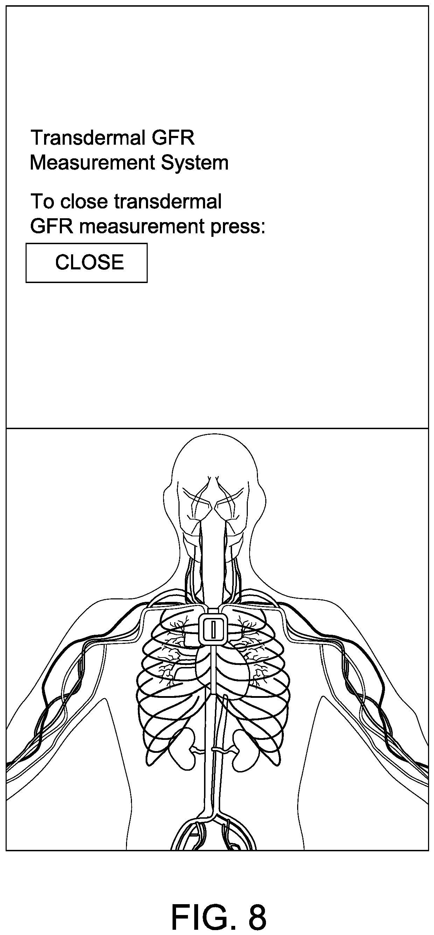

Figure 8 is an example screenshot of an exit screen for the software application. As shown in Figure 8, after completion of the assessment, the exit screen instructs the patient to close the software application. Additional instructions may be included here or on an additional screen. The patient may be instructed to remove the sensor from their body and/or how to dispose of it properly. In some aspects, the sensor may be reusable, and the patient is instructed how to properly remove and clean the sensor in preparation for reuse. Additional instructions may include the final disposition of the injector device and or dose cartridge of the GFR agent.

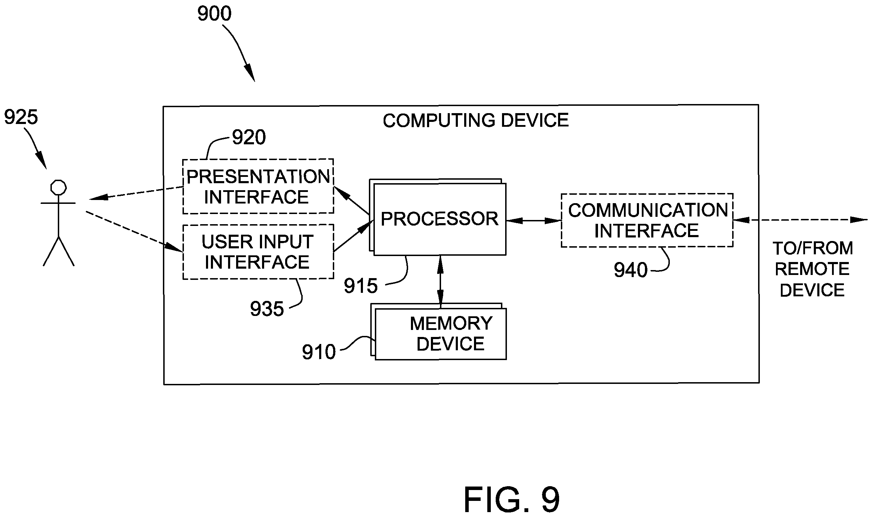

Figure 9 is a block diagram of one embodiment of a computing device 900 that may be used to implement the mobile computing device operating the software application described herein. For example, computing device 900 may facilitate performing at least some of the functions described above.

Computing device 900 includes at least one memory device 910 and a processor 915 that is coupled to memory device 910 for executing instructions. In some embodiments, executable instructions are stored in memory device 910. Computing device 900 performs one or more operations described herein by programming processor 915. For example, processor 915 may be programmed by encoding an operation as one or more executable instructions and by providing the executable instructions in memory device 910.

Processor 915 may include one or more processing units (e.g., in a multi-core configuration). Further, processor 915 may be implemented using one or more heterogeneous processor systems in which a main processor is present with secondary processors on a single chip. As another illustrative example, processor 915 may be a symmetric multi-processor system containing multiple processors of the same type. Further, processor 915 may be implemented using any suitable programmable circuit including one or more systems and microcontrollers, microprocessors, reduced instruction set circuits (RISC), application specific integrated circuits (ASIC), programmable logic circuits, field programmable gate arrays (FPGA), and any other circuit capable of executing the functions described herein.

Memory device 910 is one or more devices that enable information such as executable instructions and/or other data to be stored and retrieved. Memory device 910 may include one or more computer readable media, such as, without limitation, dynamic random access memory (DRAM), static random access memory (SRAM), a solid state disk, and/or a hard disk. The memory device 910 may be configured to store, without limitation, application source code, application object code, source code portions of interest, object code portions of interest, configuration data, execution events and/or any other type of data.

Computing device 900 includes a presentation interface 920 that is coupled to processor 915. Presentation interface 920 presents information to a user 925, such as the patient. For example, presentation interface 920 may include a display adapter (not shown) that may be coupled to a display device, such as a cathode ray tube (CRT), a liquid crystal display (LCD), an organic LED (OLED) display, and/or an "electronic ink" display. In some embodiments, presentation interface 920 includes one or more display devices.

In the embodiment shown in Figure 9, computing device 900 includes a user input interface 935. In this embodiment, user input interface 935 is coupled to processor 915 and receives input from user 925. User input interface 935 may include, for example, a keyboard, a pointing device, a mouse, a stylus, a touch sensitive panel (e.g., a touch pad or a touch screen), a gyroscope, an accelerometer, a position detector, and/or an audio user input interface. A single component, such as a touch screen, may function as both a display device of presentation interface 920 and user input interface 935.

Computing device 900 includes a communication interface 940 coupled to processor 915 in this embodiment. Communication interface 940 communicates with one or more remote devices. To communicate with remote devices, communication interface 940 may include, for example, a wired network adapter, a wireless network adapter, and/or a mobile telecommunications adapter.

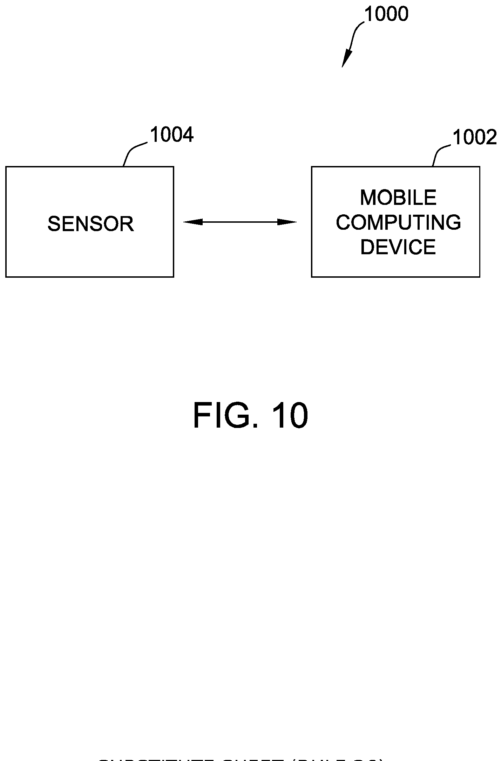

Figure 10 is a block diagram of a system 1000 for assessing the GFR in a patient. As illustrated in Figure 10, the system generally includes a mobile computing device 1002 (e.g., computing device 900 (shown in Figure 9)) communicatively coupled to a GFR sensor 1004. As described above, mobile computing device 1002 executes a software application that assists a user in assessing GFR in the patient using GFR sensor 1004. In some embodiments, disclosed herein is a non-transitory computer-readable media having computer-executable instructions thereon. When the instructions are executed by at least one processor of a mobile computing device it causes the at least one processor of the mobile computing device to display a pairing screen that instructs a user to wirelessly communicatively couple the mobile computing device to a GFR sensor, display a sensor placement screen that instructs the user to place the GFR sensor on a body of a patient, display an injection screen that instructs the user to administer a GFR agent into the body of the patient, transmit a signal from the mobile computing device to the GFR sensor to cause the GFR sensor to initiate collection of light absorbance data for calculating a GFR of the patient, receive light absorbance data from the GFR sensor, and store the received light absorbance data.

The system may optionally comprise additional components. For example, the system for assessing the GFR in a patient may further comprise a sensor. In some aspects, the sensor is as described elsewhere herein. In some aspects, the system may further comprise an injector for the GFR agent. In some aspects, the injector and the GFR agent are as described elsewhere herein.

In some aspects, the instructions also cause the at least one processor to calculate the GFR of the patient using the light absorbance data. In some aspects, the instructions further cause the at least one processor to wirelessly transmit the GFR of the patient to a computing device of a health care provider. In some aspects, the instructions further cause the at least one processor to the display a customization screen that instructs the patient to calibrate the GFR sensor on the body of the patient, to transmit a signal from the mobile computing device to the GFR sensor to initiate calibration of the GFR sensor in response to receiving a user input to initiate sensor calibration, and to receive a signal from the GFR sensor upon completion of sensor calibration. In some aspects, the computer-executable instructions further cause the processor of the mobile computing device to display an error message if an error occurs during execution of the instructions.

In still yet another aspect, disclosed herein is a computer-implemented method for assessing the GFR in a patient in need thereof. The method is implemented using a mobile computing device that comprises at least one processor in communication with at least one memory, and at least one user interface, and the method generally includes displaying a sensor placement screen that instructs the user to place a sensor on the body of the patient, displaying a pairing screen that instructs the user on how to communicatively couple the sensor on the body of the patient to the mobile computing device, displaying an injection screen that instructs the user on how/where to administer a GFR agent into the body of the patient, displaying an measurement screen that instructs the user on how to initiate collection of light absorbance data by the sensor, displaying a collection screen that instructs the user to wait a predetermined period of time while the sensor collects light absorbance data, and displaying a transmission screen that instructs the user on how to transmit the light absorbance data to a computing device of a health care provider.

In some aspects, the method further includes one or more of the following additional steps: displaying a customization screen that instructs the user on how to customize the sensor prior to collecting light absorbance data, calculating the GFR of the patient using the light absorbance data, displaying a results screen that displays the calculated GFR of the patient, waiting for patient/user input after each step in the computer implemented method indicating that the step was successfully completed, transmitting from the mobile computing device to a computing device of a health care provider the calculated GFR, the light absorbance data, or both, and/or providing feedback to the user and/or patient if an error occurs at any time during execution of the method.

In still yet another aspect, disclosed herein is a kit for GFR assessment. The kit generally includes an injector device configured to administer a GFR agent into the body of a patient, the GFR agent configured to emit at least one response light in response to the electromagnetic radiation generated by the sensor, and be eliminated by glomerular filtration by the kidneys of the patient; a sensor configured to attach to the body of the patient, emit electromagnetic radiation in the direction of the body of the patient, and detect at least one response light emitted by the GFR agent inside the body of the patient in response to the electromagnetic radiation, a mobile computing device wirelessly communicatively coupled to the sensor and programmed to receive response light data from the sensor, and calculate the GFR of the patient based on the response light data. In some aspects, the GFR agent is as described elsewhere herein. In some aspects, the sensor is as described elsewhere herein. In some aspects, the injector device is as described elsewhere herein. In some aspects, the mobile computing device is as described elsewhere herein.

In some aspects, the kit further comprises written instructions describing how to use the components of the kit in order to assess the GFR of the patient.

As used herein, the term "patient" and "user" may or may not refer to the same person. In some aspects, they are the same. In some aspects, they are different. When they are different, the user of the computer program is assisting the patient with the GFR assessment. For example, a home health nurse may assist a patient with the GFR assessment in the patient's own home thus saving the patient the difficulty of travelling to a doctor's office or hospital in order to have a GFR assessment performed. It is understood that the patient can be male or female, and that gendered pronouns used herein are used simply as a linguistic convenience.

Examples of GFR agents, sometimes called indicator substances, suitable for use with the methods and devices herein include, but are not limited to, those disclosed in US 62/577,951, US 8,155,000, US 8,664,392, US 8,697,033, US 8,722,685, US 8,778,309, US 9,005,581, US 9,114,160, US 9,283,288, US 9,376,399, and US 9,480,687. In some aspects, the indicator substance is eliminated from the body of a patient by glomerular filtration. In some aspects, the indicator substance is eliminated from the body of a patient only by glomerular filtration.

In some aspects, the indicator substance is a pyrazine derivative of Formula I, or a pharmaceutically acceptable salt thereof, 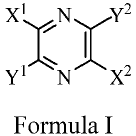 wherein each of X¹ and X² is independently selected from the group consisting of -CN, -CO₂R¹, -CONR¹R², -CO(AA), -CO(PS) and -CONH(PS); each of Y¹ and Y² is independently selected from the group consisting of -NR¹R² and 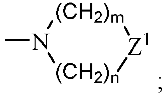 Z1 is a single bond, -CR1R2-, -O-, -NR1-, -NCOR1-, -S-, -SO-, or -SO2-; each of R1 to R2 are independently selected from the group consisting of H, -CH2(CHOH)aH, -CH2(CHOH)aCH3, -CH2(CHOH)aCO2H, -(CHCO2H)aCO2H, -(CH2CH2O)cH, -(CH2CH2O)cCH3, -(CH2)aSO3H, -(CH2)aSO3-, -(CH2)aSO2H, -(CH2)aSO2-, -(CH2)aNHSO3H, -(CH2)aNHSO3-, -(CH2)aNHSO2H, -(CH2)aNHSO2-, -(CH2)aPO4H3, -(CH2)aPO4H2-, -(CH2)aPO4H2-, -(CH2)aPO43-, -(CH2)aPO3H2, -(CH2)aPO3H-, and -(CH2)aPO32-; (AA) comprises one or more amino acids selected from the group consisting of natural and unnatural amino acids, linked together by peptide or amide bonds and each instance of (AA) may be the same or different than each other instance; (PS) is a sulfated or non-sulfated polysaccharide chain that includes one or more monosaccharide units connected by glycosidic linkages; and 'a' is a number from 0 to 10, 'c' is a number from 1 to 100, and each of 'm' and 'n' are independently a number from 1 to 3. In another aspect, 'a' is a number from 1 to 10. In still yet another aspect, 'a' is 0, 1, 2, 3, 4, 5, 6, 7, 8, 9 or 10.

(AA) comprises one or more natural or unnatural amino acids linked together by peptide or amide bonds. The peptide chain (AA) may be a single amino acid, a homopolypeptide chain or a heteropolypeptide chain, and may be any appropriate length. In some embodiments, the natural or unnatural amino acid is an α-amino acid. In yet another aspect, the α-amino acid is a D-α-amino acid or an L-α-amino acid. In a polypeptide chain that includes two or more amino acids, each amino acid is selected independently of the other(s) in all aspects, including, but not limited to, the structure of the side chain and the stereochemistry. For example, in some embodiments, the peptide chain may include 1 to 100 amino acid(s), 1 to 90 amino acid(s), 1 to 80 amino acid(s), 1 to 70 amino acid(s), 1 to 60 amino acid(s), 1 to 50 amino acid(s), 1 to 40 amino acid(s), 1 to 30 amino acid(s), 1 to 20 amino acid(s), or even 1 to 10 amino acid(s). In some embodiments, the peptide chain may include 1 to 100 α-amino acid(s), 1 to 90 α-amino acid(s), 1 to 80 α-amino acid(s), 1 to 70 α-amino acid(s), 1 to 60 α-amino acid(s), 1 to 50 α-amino acid(s), 1 to 40 α-amino acid(s), 1 to 30 α-amino acid(s), 1 to 20 α-amino acid(s), or even 1 to 10 α-amino acid(s). In some embodiments, the amino acid is selected from the group consisting of D-alanine, D-arginine D-asparagine, D-aspartic acid, D-cysteine, D-glutamic acid, D-glutamine, glycine, D-histidine, D-homoserine, D-isoleucine, D-leucine, D-lysine, D-methionine, D-phenylalanine, D-proline, D-serine, D-threonine, D-tryptophan, D-tyrosine, and D-valine. In some embodiments, the α-amino acids of the peptide chain (AA) are selected from the group consisting of arginine, asparagine, aspartic acid, glutamic acid, glutamine, histidine, homoserine, lysine, and serine. In some embodiments, the α-amino acids of the peptide chain (AA) are selected from the group consisting of aspartic acid, glutamic acid, homoserine and serine. In some embodiments, the peptide chain (AA) refers to a single amino acid (e.g., D-aspartic acid or D-serine).

(PS) is a sulfated or non-sulfated polysaccharide chain including one or more monosaccharide units connected by glycosidic linkages. The polysaccharide chain (PS) may be any appropriate length. For instance, in some embodiments, the polysaccharide chain may include 1 to 100 monosaccharide unit(s), 1 to 90 monosaccharide unit(s), 1 to 80 monosaccharide unit(s), 1 to 70 monosaccharide unit(s), 1 to 60 monosaccharide unit(s), 1 to 50 monosaccharide unit(s), 1 to 40 monosaccharide unit(s), 1 to 30 monosaccharide unit(s), 1 to 20 monosaccharide unit(s), or even 1 to 10 monosaccharide unit(s). In some embodiments, the polysaccharide chain (PS) is a homopolysaccharide chain consisting of either pentose or hexose monosaccharide units. In other embodiments, the polysaccharide chain (PS) is a heteropolysaccharide chain consisting of one or both pentose and hexose monosaccharide units. In some embodiments, the monosaccharide units of the polysaccharide chain (PS) are selected from the group consisting of glucose, fructose, mannose, xylose and ribose. In some embodiments, the polysaccharide chain (PS) refers to a single monosaccharide unit (e.g., either glucose or fructose). In yet another aspect, the polysaccharide chain is an amino sugar where one or more of the hydroxy groups on the sugar has been replaced by an amine group. The connection to the carbonyl group can be either through the amine or a hydroxy group.

Specific examples of indicator substances include, but are not limited to, 3,6-diamino-N2,N2,N5,N5-tetrakis(2-methoxyethyl)pyrazine-2,5-dicarboxamide, 3,6-diamino-N2,N5-bis(2,3-dihydroxypropyl)pyrazine-2,5-dicarboxamide, (2S,2'S)-2,2'-((3,6-diaminopyrazine-2,5-dicarbonyl)bis(azanediyl))bis(3-hydroxypropanoic acid), 3,6-bis(bis(2-methoxyethyl)amino)-N2,N2,N5,N5-tetrakis(2-methoxy ethyl) pyrazine-2,5-dicarboxamide bis(TFA) salt, 3,6-diamino-N2,N5-bis(2-aminoethyl)pyrazine-2,5-dicarboxamide bis(TFA) salt, 3,6-diamino-N2,N5-bis (D-aspartate)-pyrazine-2,5-dicarboxamide, 3,6-diamino-N2,N5-bis(14-oxo-2,5,8,11-tetraoxa-15-azaheptadecan-17-yl)pyrazine-2,5-dicarboxamide, 3,6-diamino-N2,N5-bis(26-oxo-2,5,8,11,14,17,20,23-octaoxa-27-azanonacosan-29-yl)pyrazine-2,5-dicarboxamide, 3,6-diamino-N2,N5-bis(38-oxo-2,5,8, 11, 14, 17,20,23,26,29,32,35-dodecaoxa-39-azahentetracontan-41-yl)pyrazine-2,5-dicarboxamide, bis(2-(PEG-5000)ethyl) 6-(2-(3,6-diamino-5-(2-aminoethylcarbamoyl) pyrazine-2-carboxamido)ethylamino)-6-oxohexane-1,5-diyldicarbamate, (R)-2-(6-(bis(2-methoxyethyl)amino)-5-cyano-3-morpholinopyrazine-2-carboxamido)succinic acid, (2R,2'R)-2,2'-((3,6-diaminopyrazine-2,5-dicarbonyl)bis(azanediyl))bis(3-hydroxypropanoic acid), (25,2'S)-2,2'-((3,6-diaminopyrazine-2,5-dicarbonyl)bis(azanediyl))bis(3-hydroxypropanoic acid), (2R,2'R)-2,2'-((3,6-diaminopyrazine-2,5-dicarbonyl)bis(azanediyl)) dipropionic acid, 3,3'-((3,6-diaminopyrazine-2,5-dicarbonyl)bis(azanediyl))dipropionic acid, 2,2'-((3,6-diaminopyrazine-2,5-dicarbonyl)bis(azanediyl))diacetic acid, (25,2'S)-2,2'-((3,6-diaminopyrazine-2,5-dicarbonyl)bis(azanediyl)) dipropionic acid, 2,2'-((3,6-diaminopyrazine-2,5-dicarbonyl)bis(azanediyl))bis(2-methylpropanoic acid), and 3,6-diamino-N2,N5-bis((1R,2S,3R,4R)-1,2,3,4,5-pentahydroxypentyl) pyrazine-2,5-dicarboxamide. In some aspects, the indicator substance is (2R,2'R)-2,2'-((3,6-diaminopyrazine-2,5-dicarbonyl)bis(azanediyl))bis(3-hydroxypropanoic acid) (also known as MB-102). In some aspects, the indicator substance is (25,2'S)-2,2'-((3,6-diaminopyrazine-2,5-dicarbonyl)bis(azanediyl))bis(3-hydroxypropanoic acid).

In some aspects, the indicator substance is (2R,2'R)-2,2'-((3,6-diamino-pyrazine-2,5-dicarbonyl)bis(azanediyl))bis(3-hydroxypropanoic acid) (also known as MB-102 or 3,6-diamino-N2,N5-bis(D-serine)-pyrazine-2,5-dicarboxamide), 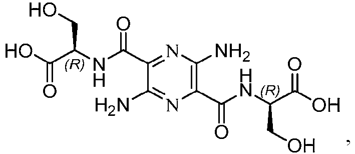 or a pharmaceutically acceptable salt thereof.

In some aspects, the indicator substance is (2S,2'S)-2,2'-((3,6-diamino-pyrazine-2,5-dicarbonyl)bis(azanediyl))bis(3-hydroxypropanoic acid) (also known as 3,6-diamino-N2,N5-bis(L-serine)-pyrazine-2,5-dicarboxamide), 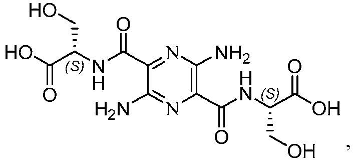 or a pharmaceutically acceptable salt thereof.

In still yet another aspect, the indicator substance is selected from the group consisting of acridines, acridones, anthracenes, anthracylines, anthraquinones, azaazulenes, azo azulenes, benzenes, benzimidazoles, benzofurans, benzoindocarbocyanines, benzoindoles, benzothiophenes, carbazoles, coumarins, cyanines, dibenzofurans, dibenzothiophenes, dipyrrolo dyes, flavones, imidazoles, indocarbocyanines, indocyanines, indoles, isoindoles, isoquinolines, naphthacenediones, naphthalenes, naphthoquinones, phenanthrenes, phenanthridines, phenanthridines, phenoselenazines, phenothiazines, phenoxazines, phenylxanthenes, polyfluorobenzenes, purines, pyrazines, pyrazoles, pyridines, pyrimidones, pyrroles, quinolines, quinolones, rhodamines, squaraines, tetracenes, thiophenes, triphenyl methane dyes, xanthenes, xanthones, and derivatives thereof. In still yet another aspect, the indicator substance is any compound that is eliminated from the body of a patient by glomerular filtration. In still yet another aspect, the indicator substance is any compound that emits fluorescent energy when exposed to electromagnetic radiation and is eliminated from the body of the patient by glomerular filtration.

In any aspect of the indicator substance, one or more atoms may alternatively be substituted with an isotopically labelled atom of the same element. For example, a hydrogen atom may be isotopically labelled with deuterium or tritium; a carbon atom may be isotopically labelled with ¹³C or ¹⁴C; a nitrogen atom may be isotopically labelled with ¹⁴N or ¹⁵N. An isotopic label may be a stable isotope or may be an unstable isotope (i.e., radioactive). The indicator substance may contain one or more isotopic labels. The isotopic label may be partial or complete. For example, an indicator substance may be labeled with 50% deuterium thereby giving the molecule a signature that can be readily monitored by mass spectroscopy or other technique. As another example, the indicator substance may be labeled with tritium thereby giving the molecule a radioactive signature that can be monitored both *in vivo* and *ex vivo* using techniques known in the art.

Pharmaceutically acceptable salts are known in the art. In any aspect herein, the indicator substance may be in the form of a pharmaceutically acceptable salt. By way of example and not limitation, pharmaceutically acceptable salts include those as described by Berge, et al. in J. Pharm. Sci., 66(1), 1 (1977). The salt may be cationic or anionic. In some embodiments, the counter ion for the pharmaceutically acceptable salt is selected from the group consisting of acetate, benzenesulfonate, benzoate, besylate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methyl sulfate, mucate, napsylate, nitrate, pamoate, pantothenate, phosphate, diphosphate, polygalacturonate, salicylate, stearate, subacetate, succinate, sulfate, tannate, tartrate, teoclate, triethiodide, adipate, alginate, aminosalicylate, anhydromethylenecitrate, arecoline, aspartate, bisulfate, butylbromide, camphorate, digluconate, dihydrobromide, disuccinate, glycerophosphate, jemisulfate, judrofluoride, judroiodide, methylenebis(salicylate), napadisylate, oxalate, pectinate, persulfate, phenylethylbarbarbiturate, picrate, propionate, thiocyanate, tosylate, undecanoate, benzathine, chloroprocaine, choline, diethanolamine, ethylenediamine, meglumine, procaine, benethamine, clemizole, diethylamine, piperazine, tromethamine, aluminum, calcium, lithium, magnesium, potassium, sodium zinc, barium and bismuth. Any functional group in the indicator substance capable of forming a salt may optionally form one using methods known in the art. By way of example and not limitation, amine hydrochloride salts may be formed by the addition of hydrochloric acid to the indicator substance. Phosphate salts may be formed by the addition of a phosphate buffer to the indicator substance. Any acid functionality present, such as a sulfonic acid, a carboxylic acid, or a phosphonic acid, may be deprotonated with a suitable base and a salt formed. Alternatively, an amine group may be protonated with an appropriate acid to form the amine salt. The salt form may be singly charged, doubly charged or even triply charged, and when more than one counter ion is present, each counter ion may be the same or different than each of the others.

The injector device is configured such that a patient is able to self-administer the GFR agent outside of a hospital or clinical setting. For example, the patient is able to administer the GFR agent while at home. In some aspects, the injector device comes preloaded with the GFR agent already loaded into the device. In some aspects, the GFR agent is in a dose cartridge or other container, and the patient is provided with instructions as to how to load the dose cartridge or container into the injector device. In some aspects, the injector device is designed so that the patient can self-administer the GFR agent directly into their circulatory system, into their lymphatic system, into the subdermal space, or subcutaneously. In some aspects, the patient is able to self-administer the GFR agent through the vessels in the mouth. In some aspects, the GFR agent is administered orally.

The sensor (also referred to as a "GFR sensor") comprises at least one radiation source. A radiation source is understood to be any device which can emit radiation anywhere on the electromagnetic spectrum. In some aspects, the electromagnetic radiation is in the visible, infrared, ultraviolet, and/or gamma spectral range. Without restricting the type of radiation used and for convenience only, hereinafter radiation is generally designated as "light" whether or not it is in the visible region of the electromagnetic spectrum, and the radiation source is described more particularly with reference to a "light source". However, other configurations of the radiation source are possible, in some aspects, and it is also possible, in some aspects, to combine different types of radiation sources.

The radiation source can be, for example, an integral constituent of the sensor, for example in the context of a layer construction of the sensor. The radiation source is therefore designed to generate at least one interrogation light directly within the sensor, in contrast to external generation of the interrogation light. Instead of an individual light source, in some aspects, it is also possible to use a plurality of light sources, for example redundant light sources for emitting one and the same wavelength, and/or a plurality of different light sources for emitting different wavelengths. Generally, the at least one light source is designed to irradiate the body surface with at least one interrogation light.

An interrogation light is understood to be a light that can be used for the detection of an indicator substance as disclosed elsewhere herein, whose light excites the indicator substance inside a body tissue and/or a body fluid of the patient, for example with variable penetration depth, and causing a perceptible response, more particularly, an optically perceptible response. This excitation takes place in such a way that a luminescence, a fluorescence and/or a phosphorescence is initiated in the indicator substance. In some aspects, other types of excitation occur, for example scattering of the light at an identical or shifted wavelength. Generally, at least one response light is generated by the indicator substance in response to the interrogation light.

The interrogation light is designed such that the desired response is excited in a targeted manner in the indicator substance. Accordingly, by way of example and not limitation, a wavelength and/or a wavelength range of the interrogation light and/or some other property of the interrogation light can be adapted or adjusted based on the identity and properties of the indicator substance. This can be done directly by the radiation source, for example, by virtue of the radiation source providing the interrogation light having a specific wavelength and/or in a specified wavelength range and/or by the inclusion of at least one excitation filter being used to filter out the desired interrogation light from a primary light of the light source. In some aspects, the sensor performs fluorescence measurements on the indicator substance. Accordingly, the interrogation light can be adapted to the excitation range of the fluorescence of the indicator substance.

The sensor further comprises at least one detector designed to detect at least one response light incident from the direction of the body surface. The response light can be light in the sense of the above definition. The detector is also an integral constituent of the sensor. The detector is therefore part of the sensor such that the response light is detected directly within the sensor. In some aspects, the detector is configured for diffuse reflection correction such that any light that does not emanate directly from the GFR agent inside the body of the patient can be either filtered out or corrected by way of background correction.

In some aspects, the response light represents an optical response of the indicator substance to the incidence of the interrogation light. Accordingly, the detector and/or the detector in interaction with at least one response filter is configured to detect in a targeted manner in the spectral range of the response light. In some aspects, the detector and/or the detector in interaction with the at least one response filter is configured to suppress light outside the spectral range of the response light. In some aspects, the detector and/or the detector in interaction with the at least one response filter can be designed to suppress the interrogation light. In yet another aspect, response filters are designed to suppress the detection of ambient light, particularly at wavelengths that can travel long distances in tissue prior to absorption, such as a spectral range of from about 700 to about 1100 nm. The interrogation light and the response light can be configured such that they are spectrally different or spectrally shifted relative to one another with regard to their spectral intensity distribution.

By way of example and not limitation, in some aspects, the response light shifts toward longer wavelengths in comparison with the interrogation light, which generally occurs in a fluorescence measurement (i.e., the Stokes shift). By way of another example, the Stokes shift of a peak wavelength of the response light relative to a peak wavelength of the interrogation light is between about 10 nm and about 200 nm, more particularly between about 100 nm and about 150 nm, and particularly about 120 nm. The detector and/or the detector in interaction with the at least one response filter can be designed to detect such response light. About in this context means ± 10 nm.

The at least one radiation source, more particularly, the at least one light source, and the at least one detector are designed to irradiate the body surface with the interrogation light and to detect at least one response light incident from the direction of the body surface. The radiation source and the detector are therefore optically connected to the body surface in such a way that, through the body surface, for example transcutaneously, the interrogation light can be radiated into the body tissue or the body fluid of the patient, and that, likewise through the body surface, for example transcutaneously, the response light from the body tissue or the body fluid is observed by the detector.

In addition to the at least one detector and the at least one radiation source, the sensor assembly may comprise further elements. In some aspects, the sensor comprises further elements. Thus, the sensor can comprise, for example, at least one interface for data exchange. Said data can be, for example, measurement results for intensities of the response light detected by the detector. Data already partly processed, filtered or partly or completely evaluated data, can also be transmitted wirelessly to the computer program on the mobile computing device. In some aspects, transponder technology known in the art may be used, for example, to initiate a measurement via the sensor and/or to interrogate measurement data from the sensor. In some aspects, corresponding radiofrequency readers such as are known from RFID technology (radiofrequency identification label technology), for example, can be used for this purpose. In some aspects, Bluetooth technology is use for this purpose.

In some aspects, a 2-sided adhesive is employed as a constituent part of the sensor. The side facing the skin is selected to adhere reliably to the skin for an extended period of time (e.g., 24 to 48 hrs.), even in the presence of moisture, such as sweat. In some aspects, an acrylate-based adhesive is used for bonding to the skin. In yet another aspect, the skin is pre-treated with a barrier film, such as by application of rapidly-drying liquid film that upon drying forms a "second skin". In such aspects the barrier film aids in the long-term, reliable attachment of the acrylate-based adhesive to the skin, while also having the benefit of allowing sensor removal without disruption or removal of the skin epidermis. In some aspects, the barrier film is CAVILON^{™} (manufactured by 3M). The second side of the adhesive, which faces towards the sensor, may be selected to adhere as strongly as desired to the face of the sensor. In one such aspect the sensor face is constructed from a polymer material, such as MAKROLON^{™}, and the adhesive is rubber based. One non-limiting example of an appropriate 2-sided adhesive is 3M product # 2477 (Double-Coated TPE Silicone Acrylate Medical Tape with Premium Liner).

As used herein, an element or step recited in the singular and preceded by the word "a" or "an" should be understood as not excluding plural elements or steps, unless such exclusion is explicitly recited. Furthermore, references to "example embodiment" or "one embodiment" of the present disclosure are not intended to be interpreted as excluding the existence of additional embodiments that also incorporate the recited features.

This written description uses examples to disclose the invention, including the best mode, and also to enable any person skilled in the art to practice the invention, including making and using any devices or systems and performing any incorporated methods. The patentable scope of the invention is defined by the claims.

## Claims

1. A non-transitory computer-readable media (910) having computer-executable instructions thereon, wherein when executed by at least one processor (915) of a mobile computing device (900; 1002), cause the at least one processor (915) of the mobile computing device (900; 1002) to:
display a pairing screen (920) that instructs a use to wirelessly communicatively couple the mobile computing device (900; 1002) to the GFR sensor (1004);
display a sensor placement screen (920) that instructs the user to place the GFR sensor (1004) on a body of a patient;
display a customization screen (920) that instructs the patient to customize the sensor (1004) to a skin tone of the patient;
customize the sensor (1004) to the skin tone of the patient by collecting with the sensor a background level of light absorbance that naturally emanates from the body of the patient for customization of the sensor (1004) and adjusting a light strength in the sensor or a detector sensitivity in the sensor (1004) to account for differing skin colors of different patients;
display an injection screen (920) that instructs the user to administer a GFR agent into the body of the patient;
transmit a signal from the mobile computing device (900; 1002) to the GFR sensor (1004) to cause the GFR sensor (1004) to initiate collection of light absorbance data for calculating a GFR of the patient;
receive light absorbance data from the GFR sensor (1004); and
store the received light absorbance data.

2. The non-transitory computer-readable media (910) of claim 1, wherein the computer-executable instructions further cause the processor (915) of the mobile computing device (900; 1002) to:
calculate the GFR of the patient using the light absorbance data.

3. The non-transitory computer-readable media (910) of claim 2, wherein the computer-executable instructions further cause the processor (915) of the mobile computing device (900; 1002) to:
wirelessly transmit the GFR of the patient to a computing device of a health care provider.

4. The non-transitory computer-readable media (910) of claim 1, wherein the computer-executable instructions further cause the processor (915) of the mobile computing device (900; 1002) to:
display an error message if an error occurs during execution of the instructions.

5. A computer-implemented method for assessing a GFR in a patient in need thereof, the method implemented using a mobile computing device (900; 1002) that comprises at least one processor (915) in communication with at least one memory, and at least one user interface (920), the method comprising:
displaying a sensor placement screen (920) that instructs the user to place a sensor on the body of the patient;
displaying a pairing screen (920) that instructs the user on how to communicatively couple the sensor (1004) on the body of the patient to the mobile computing device (900; 1002);
displaying a customization screen (920) that instructs the patient to customize the sensor (1004) to a skin tone of the patient;
customizing the sensor (1004) to the skin tone of the patient by collecting a background level of light absorbance that naturally emanates from the body of the patient for customization of the sensor (1004) and adjusting a light strength or a detector sensitivity in the sensor (1004) to account for differing skin colors of different patients;
displaying an injection screen (920) that instructs the user on how/where to administer a GFR agent into the body of the patient;
displaying a measurement screen (920) that instructs the user on how to initiate collection of light absorbance data by the sensor (1004);
displaying a collection screen (920) that instructs the user to wait a predetermined period of time while the sensor (1004) collects light absorbance data; and
displaying a transmission screen that instructs the user on how to transmit the light absorbance data to a computing device of a health care provider.

6. The computer-implemented method according to claim 5, wherein the method further comprises:
calculating the GFR of the patient using the light absorbance data.

7. The computer-implemented method according to claim 6, wherein the method further comprises:
displaying a results screen (920) that displays the calculated GFR of the patient.

8. The computer-implemented method according to claim 5, wherein the method further comprises:
waiting for patient/user input after each step in the computer implemented method indicating that the step was successfully completed.

9. The computer-implemented method according to claim 5, wherein the method further comprises:
transmitting from the mobile computing device (900; 1002) to the computing device of the health care provider the calculated GFR, the light absorbance data, or both.

10. The computer-implemented method according to claim 5, wherein the method further comprises:
providing feedback to the user and/or patient if an error occurs at any time during execution of the method.

11. A kit for GFR assessment, the kit comprising:
an injector device configured to administer a GFR agent into the body of a patient;
the GFR agent configured to:
emit at least one response light in response to the electromagnetic radiation generated by the sensor; and
be eliminated by glomerular filtration by the kidneys of the patient;
a sensor (1004) configured to:
attach to the body of the patient;
emit electromagnetic radiation in the direction of the body of the patient; and
detect at least one response light emitted by the GFR agent inside the body of the patient in response to the electromagnetic radiation;
a mobile computing device (900; 1002) configured to be wirelessly communicatively coupled to the sensor (1004), wherein the mobile computing device (900; 1002) comprises at least one processor (915) in communication with at least one memory (910), and at least one user interface (920), and programmed to:
receive response light data from the sensor (1004); and
calculate the GFR of the patient based on the response light data,
wherein the processor (915) of the mobile computing device (900; 1002) is configured to
display a pairing screen (920) that instructs a user to wirelessly communicatively couple the mobile computing device (900; 1002) to the sensor (1004);
display a sensor placement screen (920) that instructs the user to place the sensor on the body of the patient;
display a customization screen (920) that instructs the patient to customize the sensor (1004) to a skin tone of the patient;
customize the sensor (1004) to the skin tone of the patient by collecting a background level of light absorbance that naturally emanates from the body of the patient for customization of the sensor (1004) and adjusting a light strength or a detector sensitivity in the sensor (1004) to account for differing skin colors of different patients; display an injection screen (920) that instructs the user to administer the GFR agent into the body of the patient;
transmit a signal from the mobile computing device (900; 1002) to the GFR sensor (1004) to cause the GFR sensor (1004) to initiate collection of light absorbance data for calculating a GFR of the patient;
receive light absorbance data from the GFR sensor (1004); and
store the received light absorbance data.

12. The kit according to claim 11, further comprising:
written instructions describing how to use the components of the kit in order to assess the GFR of the patient.

## Patentansprüche

1. Nicht-transitorisches, computerlesbares Medium (910) aufweisend computerlesbare Anweisungen, die, wenn sie von mindestens einem Prozessor (915) eines mobilen Computers (900; 1002) ausgeführt werden, den Prozessor (915) des mobilen Computers (900; 1002) veranlassen, folgendes durchzuführen:
Anzeigen eines Kopplungsbildschirms (920), der einen Benutzer anleitet, das mobile Computer (900; 1002) drahtlos mit dem GFR-Sensor (1004) zu koppeln;
Anzeigen eines Sensorplatzierungsbildschirms (920), der den Benutzer anleitet, den GFR-Sensor (1004) an dem Körper eines Patienten zu platzieren;
Anzeigen eines Anpassungsbildschirms (920), der den Patienten anleitet, den Sensor (1004) an der Hautfarbe des Patienten anzupassen;
Anpassen des Sensors (1004) an den Hautfarbe des Patienten, indem ein Hintergrundwert der Lichtabsorption gesammelt wird, der von selbst von dem Körper des Patienten kommt, um den Sensor (1004) anzupassen, und Anpassen einer Lichtstärke in dem Sensor oder einer Detektorempfindlichkeit in dem Sensor (1004), um den unterschiedlichen Hautfarben verschiedener Patienten zu entsprechen;
Anzeigen eines Injektionsbildschirms (920), der den Benutzer anleitet, ein GFR-Mittel in den Körper des Patienten zu verabreichen;
Senden eines Signals von dem mobilen Computer (900; 1002) an den GFR-Sensor (1004), um den GFR-Sensor (1004) zu veranlassen, mit der Sammlung von Lichtabsorptionsdaten zur Berechnung des GFR des Patienten zu beginnen;
Empfangen Lichtabsorptionsdaten vom GFR-Sensor (1004); und
Speichern der empfangenen Lichtabsorptionsdaten.

2. Nicht-transitorisches, computerlesbares Medium (910) nach Anspruch 1, wobei die computerlesbaren Anweisungen ferner den Prozessor (915) des mobilen Computers (900; 1002) veranlassen, folgendes durchzuführen:
Berechnen des GFR des Patienten unter Verwendung der Lichtabsorptionsdaten.

3. Nicht-transitorisches, computerlesbares Medium (910) nach Anspruch 2, wobei die computerlesbaren Anweisungen ferner den Prozessor (915) des mobilen Computers (900; 1002) veranlassen, folgendes durchzuführen:
Übertragen des GFR des Patienten drahtlos an ein Computer eines Gesundheitsdienstleisters.

4. Nicht-transitorisches, computerlesbares Medium (910) nach Anspruch 1, wobei die computerlesbaren Anweisungen ferner den Prozessor (915) des mobilen Computers (900; 1002) veranlassen, folgendes durchzuführen:
Anzeigen einer Fehlermeldung, wenn ein Fehler während der Ausführung der Anweisungen auftritt.

5. Computerimplementiertes Verfahren zum Bewerten eines GFR bei einem Patienten, der dies benötigt, wobei das Verfahren unter Verwendung eines mobilen Computers (900; 1002) implementiert wird, das mindestens einen Prozessor (915) in Kommunikation mit mindestens einem Speicher und mindestens einer Benutzerschnittstelle (920) umfasst, wobei das Verfahren umfasst:
Anzeigen eines Sensorplatzierungsbildschirms (920), der den Benutzer anleitet, einen Sensor an dem Körper des Patienten zu platzieren;
Anzeigen eines Kopplungsbildschirms (920), der den Benutzer anleitet, wie der Sensor (1004) an dem Körper des Patienten drahtlos mit dem mobilen Computer (900; 1002) gekoppelt wird;
Anzeigen eines Anpassungsbildschirms (920), der den Patienten anleitet, den Sensor (1004) an der Hautfarbe des Patienten anzupassen;
Anpassen des Sensors (1004) an der Hautfarbe des Patienten, indem ein Hintergrundwert der Lichtabsorption gesammelt wird, der von selbst von dem Körper des Patienten kommt, um den Sensor (1004) anzupassen, und Anpassen einer Lichtstärke oder einer Detektorempfindlichkeit in dem Sensor (1004), um unterschiedlichen Hautfarben verschiedener Patienten zu entsprechen;
Anzeigen eines Injektionsbildschirms (920), der den Benutzer anleitet, wie und wo ein GFR-Mittel in den Körper des Patienten verabreicht wird;
Anzeigen eines Messbildschirms (920), der den Benutzer anleitet, wie mit der Sammlung von Lichtabsorptionsdaten durch den Sensor (1004) zu beginnen;
Anzeigen eines Sammelbildschirms (920), der den Benutzer anleitet, eine vordefinierte Zeitspanne zu warten, während der Sensor (1004) Lichtabsorptionsdaten sammelt; und
Anzeigen eines Übertragungsbildschirms, der den Benutzer anleitet, wie die Lichtabsorptionsdaten an ein Computer eines Gesundheitsdienstleisters übertragen werden.

6. Computerimplementiertes Verfahren nach Anspruch 5, wobei das Verfahren ferner umfasst:
Berechnen des GFR des Patienten unter Verwendung der Lichtabsorptionsdaten.

7. Computerimplementiertes Verfahren nach Anspruch 6, wobei das Verfahren ferner umfasst:
Anzeigen eines Ergebnisbildschirms (920), der den berechneten GFR des Patienten anzeigt.

8. Computerimplementiertes Verfahren nach Anspruch 5, wobei das Verfahren ferner umfasst:
Warten auf eine Eingabe des Benutzers/Patienten nach jedem Schritt in dem computerimplementierten Verfahren, die anzeigt, dass der Schritt erfolgreich abgeschlossen wurde.

9. Computerimplementiertes Verfahren nach Anspruch 5, wobei das Verfahren ferner umfasst:
Übertragen des berechneten GFR, der Lichtabsorptionsdaten oder beider Werte von dem mobilen Computer (900; 1002) an das Computer des Gesundheitsdienstleisters.

10. Computerimplementiertes Verfahren nach Anspruch 5, wobei das Verfahren ferner umfasst:
Bereitstellen einer Rückmeldung an den Benutzer und/oder Patienten, wenn ein Fehler in jedem Zeitpunkt während der Ausführung des Verfahrens auftritt.

11. Kit zum Bewerten des GFR, das Kit umfassend:
ein Injektionsgerät, das konfiguriert ist, ein GFR-Mittel in den Körper eines Patienten zu verabreichen, wobei das GFR-Mittel konfiguriert ist, um:
mindestens ein Reaktionstlicht als Reaktion auf die vom Sensor erzeugte elektromagnetische Strahlung zu emittieren; und
durch glomeruläre Filtration in den Nieren des Patienten ausgeschieden zu werden;
einen Sensor (1004), der konfiguriert ist:
an dem Körper des Patienten angebracht zu werden;
elektromagnetische Strahlung in Richtung des Körpers des Patienten zu emittieren; und
mindestens ein Reaktionslicht zu erfassen, das vom GFR-Mittel in dem Körper des Patienten als Reaktion auf die elektromagnetische Strahlung emittiert wird;
ein mobiles Computer (900; 1002), das konfiguriert ist, drahtlos mit dem Sensor (1004) gekoppelt zu werden, wobei das mobile Computer (900; 1002) mindestens einen Prozessor (915) in Kommunikation mit mindestens einem Speicher (910) und mindestens einer Benutzerschnittstelle (920) umfasst und das programmiert ist, um:
Reaktionslichtdaten vom Sensor (1004) zu empfangen;
den GFR des Patienten basierend auf den Antwortlichtdaten zu berechnen,
wobei der Prozessor (915) des mobilen Computers (900; 1002) konfiguriert ist, um:
einen Kopplungsbildschirm (920) anzuzeigen, der den Benutzer anleitet, das mobile Computer (900; 1002) drahtlos mit dem Sensor (1004) zu koppeln;
einen Sensorplatzierungsbildschirm (920) anzuzeigen, der den Benutzer anleitet, den Sensor an dem Körper des Patienten zu platzieren;
einen Anpassungsbildschirm (920) anzuzeigen, der den Patienten anleitet, den Sensor (1004) an der Hautfarbe des Patienten anzupassen;
den Sensor (1004) an der Hautfarbe des Patienten anzupassen, indem ein Hintergrundwert der Lichtabsorption gesammelt wird, der von selbst von dem Körper des Patienten kommt, um den Sensor (1004) anzupassen, und eine Lichtstärke oder eine Detektorempfindlichkeit in dem Sensor (1004) anzupassen, um unterschiedlichen Hautfarben verschiedener Patienten zu entsprechen; ein Injektionsbildschirm (920) anzuzeigen, der den Benutzer anleitet, das GFR-Mittel in den Körper des Patienten zu verabreichen;
ein Signals von dem mobilen Computer (900; 1002) an den GFR-Sensor (1004) zu senden, um den GFR-Sensor (1004) zu veranlassen, mit der Sammlung von Lichtabsorptionsdaten zum Berechnen des GFR des Patienten zu beginnen;
Lichtabsorptionsdaten vom GFR-Sensor (1004) zu empfangen; und
die empfangenen Lichtabsorptionsdaten zu speichern.

12. Kit nach Anspruch 11, ferner umfassend:
schriftliche Anweisungen, die beschreiben, wie die Komponenten des Kits zu verwenden, um den GFR des Patienten zu bewerten.

## Revendications

1. Un support non transitoire lisible par ordinateur (910) comprenant des instructions exécutables par ordinateur, lesquelles, lorsqu'elles sont exécutées par au moins un processeur (915) d'un dispositif informatique mobile (900; 1002), amènent ledit au moins un processeur (915) du dispositif informatique mobile (900; 1002) à:
afficher un écran d'appairage (920) qui guide un utilisateur pour coupler sans fil le dispositif informatique mobile (900; 1002) au capteur GFR (1004);
afficher un écran de placement du capteur (920) qui guide l'utilisateur pour placer le capteur GFR (1004) sur le corps d'un patient;
afficher un écran de personnalisation (920) qui guide le patient pour personnaliser le capteur (1004) en fonction du teint de la peau du patient;
personnaliser le capteur (1004) selon le teint de la peau du patient en collectant, avec le capteur, un niveau de fond d'absorbance de lumière émanant naturellement du corps du patient pour la personnalisation du capteur (1004) et en ajustant une intensité lumineuse dans le capteur ou une sensibilité du détecteur dans le capteur (1004) pour tenir compte des différences de couleur de peau entre patients;
afficher un écran d'injection (920) qui guide l'utilisateur pour administrer un agent GFR dans le corps du patient;
transmettre un signal du dispositif informatique mobile (900; 1002) au capteur GFR (1004) pour provoquer le démarrage de la collecte des données d'absorbance de lumière par le capteur GFR (1004) afin de calculer un GFR du patient;
recevoir des données d'absorbance de lumière provenant du capteur GFR (1004); et
stocker les données d'absorbance de lumière reçues.

2. Le support non transitoire lisible par ordinateur (910) selon la revendication 1, dans lequel les instructions exécutables par ordinateur amènent en outre le processeur (915) du dispositif informatique mobile (900; 1002) à:
calculer le GFR du patient à l'aide des données d'absorbance de lumière.

3. Le support non transitoire lisible par ordinateur (910) selon la revendication 2, dans lequel les instructions exécutables par ordinateur amènent en outre le processeur (915) du dispositif informatique mobile (900; 1002) à:
transmettre sans fil le GFR du patient à un dispositif informatique d'un prestataire de soins de santé.

4. Le support non transitoire lisible par ordinateur (910) selon la revendication 1, dans lequel les instructions exécutables par ordinateur amènent en outre le processeur (915) du dispositif informatique mobile (900; 1002) à:
afficher un message d'erreur si une erreur survient pendant l'exécution des instructions.

5. Un procédé mis en oeuvre par ordinateur pour évaluer un GFR chez un patient en ayant besoin, ledit procédé étant mis en oeuvre à l'aide d'un dispositif informatique mobile (900; 1002) comprenant au moins un processeur (915) en communication avec au moins une mémoire et au moins une interface utilisateur (920), le procédé comprenant:
afficher un écran de placement de capteur (920) qui guide l'utilisateur pour placer un capteur sur le corps du patient;
afficher un écran d'appairage (920) qui guide l'utilisateur sur comment coupler de manière communicative le capteur (1004) sur le corps du patient au dispositif informatique mobile (900; 1002);
afficher un écran de personnalisation (920) qui guide le patient pour personnaliser le capteur (1004) en fonction du teint de la peau du patient;
personnaliser le capteur (1004) selon le teint de la peau du patient en collectant un niveau de fond d'absorbance de lumière émanant naturellement du corps du patient pour la personnalisation du capteur (1004) et en ajustant une intensité lumineuse ou une sensibilité du détecteur dans le capteur (1004) pour tenir compte des différences de couleur de peau entre patients;
afficher un écran d'injection (920) qui guide l'utilisateur sur comment/le lieu où administrer un agent GFR dans le corps du patient;
afficher un écran de mesure (920) qui guide l'utilisateur sur la manière d'initier la collecte des données d'absorbance de lumière par le capteur (1004);
afficher un écran de collecte (920) qui guide l'utilisateur à attendre une période prédéterminée pendant que le capteur (1004) collecte les données d'absorbance de lumière; et
afficher un écran de transmission qui guide l'utilisateur sur la manière de transmettre les données d'absorbance de lumière à un dispositif informatique d'un prestataire de soins de santé.

6. Le procédé mis en oeuvre par ordinateur selon la revendication 5, dans lequel le procédé comprend en outre:
calculer le GFR du patient à l'aide des données d'absorbance de lumière.

7. Le procédé mis en oeuvre par ordinateur selon la revendication 6, dans lequel le procédé comprend en outre:
afficher un écran de résultats (920) présentant le GFR calculé du patientt.

8. Le procédé mis en oeuvre par ordinateur selon la revendication 5, dans lequel le procédé comprend en outre:
attendre une saisie de l'utilisateur/patient après chaque étape du procédé mis en oeuvre par ordinateur, indiquant que l'étape a été effectuée avec succès.

9. Le procédé mis en oeuvre par ordinateur selon la revendication 5, dans lequel le procédé comprend en outre:
transmettre, depuis le dispositif informatique mobile (900; 1002) vers le dispositif informatique du prestataire de soins de santé, le GFR calculé, les données d'absorbance de lumière ou les deux.

10. Le procédé mis en oeuvre par ordinateur selon la revendication 5, dans lequel le procédé comprend en outre:
fournir un retour d'information à l'utilisateur et/ou au patient en cas d'erreur survenant à tout moment pendant l'exécution du procédé.

11. Un kit pour l'évaluation du GFR, le kit comprenant:
un dispositif d'injection configuré pour administrer un agent GFR dans le corps d'un patient, ledit agent GFR étant configuré pour:
émettre au moins une lumière de réponse en réaction à un rayonnement électromagnétique généré par le capteur; et
être éliminé par filtration glomérulaire par les reins du patient;
un capteur (1004) configuré pour:
s'attacher au corps du patient;
émettre un rayonnement électromagnétique en direction du corps du patient; et
détecter au moins une lumière de réponse émise par l'agent GFR à l'intérieur du corps du patient en réponse au rayonnement électromagnétique;
un dispositif informatique mobile (900; 1002) configuré pour être couplé de manière communicative sans fil au capteur (1004), ledit dispositif informatique mobile (900; 1002) comprenant au moins un processeur (915) en communication avec au moins une mémoire (910) et au moins une interface utilisateur (920), et programmé pour:
recevoir des données de lumière de réponse du capteur (1004); et
calculer le GFR du patient sur la base des données de lumière de réponse;
dans lequel le processeur (915) du dispositif informatique mobile (900; 1002) est configuré pour:
afficher un écran d'appairage (920) qui guide un utilisateur pour coupler sans fil de manière communicative le dispositif informatique mobile (900; 1002) au capteur (1004);
afficher un écran de placement du capteur (920) qui guide l'utilisateur pour placer le capteur sur le corps du patient;
afficher un écran de personnalisation (920) qui guide le patient pour personnaliser le capteur (1004) en fonction du teint de la peau du patient;
personnaliser le capteur (1004) selon le teint de la peau du patient en collectant un niveau de fond d'absorbance de lumière émanant naturellement du corps du patient pour la personnalisation du capteur (1004) et en ajustant une intensité lumineuse ou une sensibilité du détecteur dans le capteur (1004) pour tenir compte des différences de couleur de peau entre patients;
afficher un écran d'injection (920) qui guide l'utilisateur pour administrer l'agent GFR dans le corps du patient;
transmettre un signal du dispositif informatique mobile (900; 1002) au capteur GFR (1004) pour provoquer le démarrage de la collecte des données d'absorbance de lumière par le capteur GFR (1004) afin de calculer un GFR du patient;
recevoir des données d'absorbance de lumière provenant du capteur GFR (1004); et
stocker les données d'absorbance de lumière reçues.

12. Le kit selon la revendication 11, comprenant en outre:
des instructions écrites décrivant comment utiliser les composants du kit pour évaluer le GFR du patient.
